# EUROPEAN PATENT APPLICATION

(11) **EP 2 158 879 A1**
(43) Date of publication of application: **03.03.2010**
(21) Application number: 08105195.5
(22) Date of filing: 01.09.2008
(51) Int. Cl.: A61F 2/38

(54) **Trial Prosthesis for total knee arthroplasty**

(71) Applicant: MMK Consulting GmbH, 9010 St. Gallen (CH)
(72) Inventor: Kuster, Markus, St. Gallen, 9010 (CH); Kuster, Maria, 9010, St. Gallen (CH)
(74) Representative: Rentsch & Partner

(57) **Abstract**

An adjustable trial knee prosthesis device comprises a femoral component (6) and a tibial component (7), adapted to interact with each other, wherein the femoral component (6) comprises a base plate part (61), adapted for the attachment to a distal end (E,F) of a femur (2); an articulation part (63), adapted for the interaction with the tibial component (7) over essentially the whole range of natural bending angles of a knee joint; and adjustment means for adjusting the translational and/or rotational displacement of the articulation part (63) in regard to the base plate part (61). In an advantageous method for positioning a knee prosthesis, in a first step a femoral component (6) of a trial knee prosthesis device with a an articulation part (63) and a base plate part (61) is mounted on a distal end (E,F) of a femur (1), and a tibial component (7) of a trial knee prosthesis device with an articulation part (72,72') and a base plate part (71) is mounted on a proximal end (D) of a corresponding tibia (2).

## Description

### Field of the Invention

The invention relates to a trial knee prosthesis device, a femoral component of a trial knee prosthesis device, a tibial component of a trial knee prosthesis device, and a method for positioning a total condylar knee prosthesis device, according to the preamble of the independent claims.

### State of the art

Total knee arthroplasty, or total knee replacement, is an operation aimed at relieving pain and disability from degenerative arthritis, particularly osteoarthritis. It comprises the replacement of the diseased joint surfaces of the knee, particularly of the articulatio tibiofemuralis, with artificial components made for example from plastic, metal or ceramics.

First knee prostheses comprising both femoral and tibial articular surface replacements were designed as simple hinges. However, due to the complex mechanics of the knee joint, such prostheses had high failure rates due to loosening or postoperative infection. Later it was recognized that the knee joint does not simply rotate on a single axis like a simple hinge, but that the femoral condyles roll and glide on the tibia, with multiple centres of rotation. Later designs of knee prostheses then either tried to reproduce such natural knee mechanics, or to govern knee motion with a suitable artificial articulation. Modern total condylar knee prostheses try to reproduce normal joint kinematics to improve the possible range of motion of the component, relying on soft tissue such as the retained collateral ligaments and posterior cruciate ligaments to regulate joint mobility and stability after surgery.

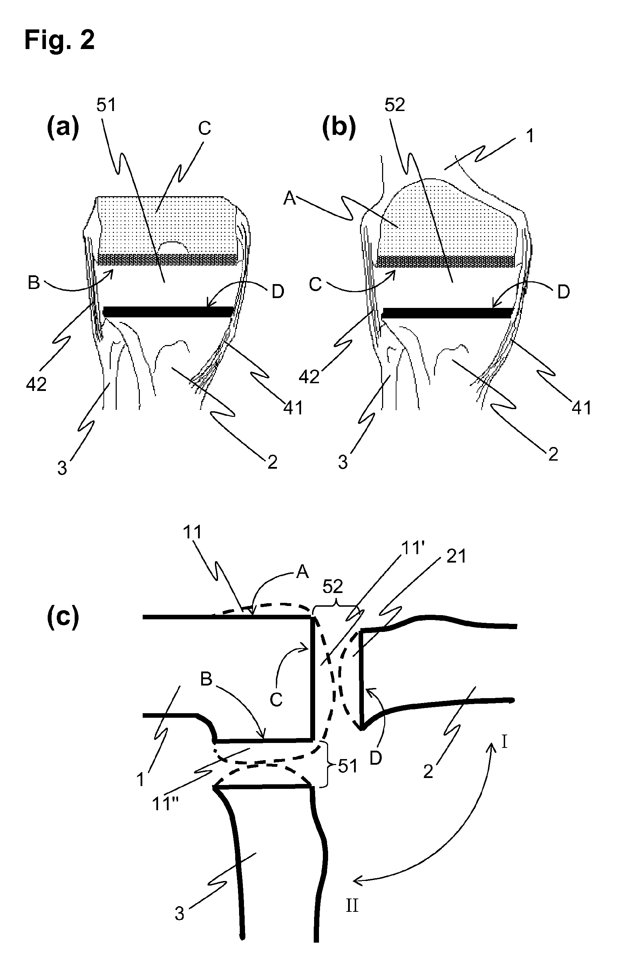

To perform total knee arthroplasty surgery the front of the knee joint has to be exposed, with detachment of part of the quadriceps muscle from the patella. The patella is displaced to one side in order to allow the exposure of the distal end of the femur bone 1 and the proximal end of the tibia bone 2. The ends of said bones 1, 2 are then cut to shape using cutting guides. Figure 2 schematically shows a knee joint after the necessary bone cuts A, B, C, D have been carried out. Figure 2(a) shows the knee joint with view onto the front side of the knee, in the flexed position II, with an angle of 90° between tibia 2 and femur 1. Figure 2(b) shows the same knee joint in the extended position I. Figure 2(c) shows a similar knee joint in a cross sectional view along the axes of femur and tibia.

To mount a standard total knee prosthesis normally 8 - 10 mm of bone material are removed at the proximal end of the tibia (cut D), 7 - 10 mm at the distal end of the femur (cut C), and 7 - 10 mm at the posterior condyles of the femur (cut B). A cutting guide is used to chamfer the edges between planes A and C, as well as planes B and C; and to produce a groove for guiding of the patella. The cartilages and the anterior cruciate ligament are removed; the collateral ligaments 41, 42 and sometimes also the posterior cruciate ligament are preserved. Metal implants are then impacted onto the bone or fixed using polymethylmethacrylate cement. The femur implant is round ended, similar to the natural shape of the femur. The tibia implant is essentially flat. To increase stability it may have a stem arranged inside the tibia. A flattened or slightly dished high density polyethylene or ceramics surface is then mounted onto the tibial implant to minimize friction. During the operation, deformities must be corrected and the remaining ligaments must be balanced so that a good range of movement is achieved, and the knee is stable. The articular surface of the patella may be replaced by a polyethylene button cemented to the posterior surface of the patella, or the patella may be retained completely.

To achieve an accurate positioning of the implants when mounting the femoral and tibial implant, different methods can be used to determine the accurate positions of the bone cuts.

With the "bone referencing" method as much bone material is removed as is later replaced by the prosthesis. Said method works quite well as long as there is no cartilage wear, bone deformation, or loss of bone tissue. The so called "ligament referencing" method on the other hand involves the determination of the positions of the bone cuts based on the amount of ligament tension. It is generally assumed for total knee arthroplasty surgery that isometric ligament tension during the whole range of motion can be achieved if the joint gaps are equal and rectangular, in both the flexion and the extension position of the knee joint. Using spreaders and force measuring devices, the rotation, the amount of bone to be removed, and the size of the prosthesis to be mounted are determined, in order to obtain equal flexion and extension gaps during surgery. This method, however, can be problematic, as several factors, such as for example femoral prosthesis size and position, can influence ligament tension and the amount of isometricity.

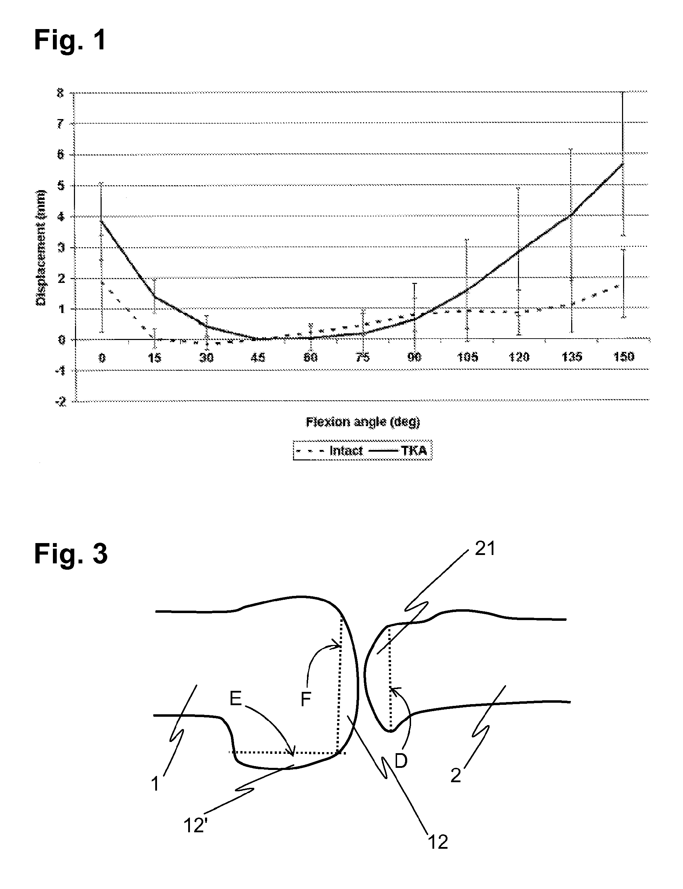

To achieve a knee motion as natural as possible, the tension of the remaining ligaments, which stabilize the knee joint prosthesis, must be in a certain range over the whole range of motion. If ligaments are too loose, the necessary overall stability of the knee is not given, and if the tension of ligaments is too high, the ligaments may be damaged, and/or the motional capabilities of the patient may remain restricted. In a recent study (R. L. Nicholls, B. O. Jeffcote, A. C. Schirm, M. S. Kuster, 2007, not yet published) it has been shown that during flexion the normal knee joint behaves almost isometrically. As can be seen in Figure 1, the experimental results clearly show that during flexion the gap remains within a range of approximately 2 mm (dotted line), while the changes of the gap width are considerably higher with a total knee arthroplasty according to the state of the art (full line).

In order to obtain a well-balanced total knee arthroplasty, with equal and rectangular flexion and extension gaps, surgeons have three possibilities for the order of bone cuts (see Figure 1). In a first variant one starts with the femur bone cuts A, B, C, and subsequently adjusts the tibia bone cut D. This so-called "femur first" technique is mainly combined with the bone referencing method. To determine the correct position of the tibial cut D, different shims may be placed under a tibial trial implant. US 2006/0111790 A1 shows a tibial trial implant with an articulation plate and a base plate connected by a double lever, which can be adjusted in height by actuating the lever mechanism.

In the other two variants, applying ligament referencing for determining the positions of the bone cuts, the proximal tibia bone cut D is carried out first ("tibia first" techniques). In a next step, the femur 1 is cut along distal plane C to obtain the intended extension gap 52, with the knee being in the extended position, tibia 2 and femur 1 being in line. Subsequently the cut along posterior plane B and the anterior plane A is carried out, adjusting the flexion gap 51 ("extension gap first" technique). With reference to Figure 2 this would mean that Figure 2(b) comes first, followed by Figure 2(a). Alternatively ("flexion first" technique) posterior cut B and anterior cut A are carried out first, with the leg being in the flexed position, thus tibia 2 and femur 1 being perpendicular to each other, as shown in Figure 2(a). The flexion gap 51 is chosen as necessary, and subsequently the cut along distal plane C is carried out to adjust the extension gap 52, as shown in Figure 2(b).

Additional cuts are carried out to further adjust the femur stump to the implant to be mounted. Preferably posterior cut B is carried out directly after the anterior cut A. The exact position and number of additional cuts depends on the type of femoral implant to be mounted. After all the bone cuts have been made, a trial prosthesis is mounted, and the knee is checked for stability and achievable range of motion. Since most or all of the bone cuts already have been carried out, the position of the prosthesis can not be changed any more and small inaccuracies must be accepted.

Irrespective of the chosen bone cut order, finding the exact balance of the extension gap 52 and the flexion gap 51 can be difficult, and various authors have used intra-operative spreaders or distraction devices to obtain equal gaps 51, 52. After the bone cuts have been made, the gap is measured at full extension I and at 90° of knee flexion II. Thus no information is obtained in the range of flexion between 0 and 90°, or beyond 90°. Furthermore several factors influence the gaps and the ligamentous stability, such as for example the size of the femur implant, rotation of the femur, joint line, etc. Although it is generally assumed that if the flexion gap 51 and the extension gap 52 are equal and rectangular, a total knee prosthesis will be well balanced, and will behave isometrically throughout the whole range of flexion, the best position and orientation of the prosthesis component may differ amongst individuals. Another problem in current knee arthroplasty is that the knee joint stability is not measured, but merely manually checked by the surgeon at the end of the operational procedure.

According to current research results more than 40% of early total knee arthroplasty revisions are due to a malpositioning of the components, incorrect size of the femoral prosthesis component, instability, or restricted range of motion (P. F. Sharkey et al., Clin. Orthop. Relat. Res., vol. 404, 2002, pp. 7-13). Most of these revisions could be avoided if the first implant would be positioned as accurate as possible.

WO 98/52499 A1 discloses a femoral trial implant, in which an articulation part can be exchanged, and can be adjusted along the proximodistal axis in regard to a base part, mounted in an existing excavation of an older femoral implant. Such a trial device is thus suitable for revisions of existing artificial knee implants. It is however not suitable as a trial prosthesis for the adjustment of a first final prosthesis, since it as bulky as a final total condylar prosthesis device.

### Objects of the Invention

One object of the invention is to provide a trial knee prosthesis device, and a femoral and a tibial component of a trial knee prosthesis device, which do not have the above-mentioned and other disadvantages, and particularly allow the determination of the isometricity of the motion of an artificial knee joint before the final, irreversible bone cuts are carried out.

Another object of the invention is to provide a trial knee prosthesis device, and a femoral and a tibial component of a trial knee prosthesis device, which allow the positioning of a final total condylar knee prosthesis device such that knee motion is as isometric as possible, in order to reduce the number of post-operational revisions due to malpositioning.

Yet another object of the invention is to provide a trial knee prosthesis device, and a femoral and a tibial component of a trial knee prosthesis device, which allow the measurement of the ligament tension over the whole natural range of flexion of a knee joint.

A further object of the invention is to provide a method for positioning a total condylar knee prosthesis device that allows to determine the most appropriate position and orientation of the prosthesis, and the suitable prosthesis size, such that an isometric knee joint motion results.

Another object of the invention is to provide a method for positioning a total condylar knee prosthesis device that reduces the risk of inadvertently removing to much femur bone material.

These and other objects are achieved by a trial knee prosthesis device, a femoral and a tibial component of a trial knee prosthesis device, and a method for positioning a total condylar knee prosthesis device, according to the independent claims. Advantageous embodiments and variants are given in the dependent claims.

### Summary of the invention

Trial knee prosthesis devices and femoral and tibial components of trial knee prosthesis devices according to the invention are adapted for the application with the tibia first techniques, in combination with the extension gap first method.

In a first step the final tibia bone cut is made. Then a small, conservative amount of bone material is removed at the distal end of the femur and at the posterior condyle. A suitable value is for example the removal of 5 mm of bone material. Now the femoral and the tibial component of the trial knee prosthesis device according to the invention are mounted.

The femoral component of the inventive trial knee prosthesis device has considerably slimmer dimensions than the femoral trial implants, and the final femoral implants known from the state of the art. The tibial component has the dimensions of the final implant. It comprises two parts, a base plate part, to be mounted on the proximal cut plane of the tibia, and an articulation part, which will interact with the femoral component. A spring or a similar resilient element is incorporated between the two parts of the tibial component to generate a repulsive force, pressing the articulation part against the distal surface of the femoral component. The motion of the resilient element or the articulation part, and/or the distance between the base plate part and the articulation part can be measured, for example with a caliper or by suitable electronic or optical means. In addition, the force acting on the resilient element, i.e. the spring force, may measured by force sensors, e.g. piezo sensors. Alternatively it is also possible to measure only the force produced by the ligaments, although this method is less precise, and furthermore includes the danger of damaging the ligaments if the tension is too high. The positions of the bones and different parts of the components of the trial prosthesis may also be determined with a surgical navigation system. An example of such a system is shown in US 2002/0133175 A1.

The surgeon will move the knee joint through the complete range of motion. Since the resilient element of the tibial component of the device according to the invention counteracts with the tension of the ligaments, the result is a constant ligament tension over the whole range of flexion of the knee joint. The isometricity of this motion is measured as a function of the flexion angle. The deviation from ideal isometricity should preferably remain within a range of approx. 2 mm around a certain average value. Hence a misplacement of the femoral trial implant will result in an increased motion of the resilient element and the articulation part. The tibial component may allow the correction of quite large deviations, for example up to 5 mm. If the deviation is even larger, the ligaments will either loosen, the force acting on the resilient element dropping to zero, or the ligaments will be overstretched, the force reaching very high values. The joint may even be blocked. In such a case adjustment shims may be placed or removed under the tibial component or the femoral component.

If the trial prosthesis already behaves isometrically, the ideal position of the prosthesis is found, and the final, irreversible bone cuts can be carried out. If the trial prosthesis is not yet moving isometrically, the following adjustments to the femoral component can be made:
a) distal and proximal translation, which affects the extension gap
b) increasing or decreasing the size of the femoral component, which affects the flexion gap
c) anterior and posterior translation, which affects the flexion gap
d) rotation in the flexion state, which affects the flexion gap
e) varus-valgus alignment, which affects the extension gap

Adjustments are repeated until an isometric motion within the accepted range of deviation is achieved. Only then, when the definite position and orientation of the final prosthesis and its appropriate size are determined, the final femur bone cuts are carried out, and the final implant is mounted.

### Brief description of the drawings

Exemplary embodiments of the invention will be described by means of figures:
- Figure 1: shows experimentally obtained values for the joint gap of an intact knee, and a knee with total knee arthroplasty, as a function of the bending angle. The fully extended knee corresponds to 0°.
- Figure 2: schematically shows a knee joint after the necessary bone cuts for total knee arthroplasty have been carried out, (a) in the flexed position II, with an angle of 90° between tibia and femur, (b) in the extended position I, and (c) in a cross sectional view parallel to the axes of femur and tibia.
- Figure 3: schematically shows an extended knee joint in a cross-sectional view along the axes of the femur and tibia bones, with the positions of the bone cuts for mounting a trial knee prosthesis device according to the invention.
- Figure 4: schematically shows a knee joint with a possible embodiment of a trial knee prosthesis device according to the invention, (a) in a lateral view, and (b) in a frontal (distal) view.
- Figure 5: schematically shows (a) a base plate part of a trial knee prosthesis device according to the invention as shown in Figure 4, and (b) a closer lateral view of the trial prosthesis in Figure 4.
- Figure 6: schematically shows two embodiments of a tibial component of a trial knee prosthesis device according to the invention.
- Figure 7: shows a further advantageous embodiment of a base plate for a femoral component of a trial prosthesis according to the invention.
- Figure 8: shows a side-view of two advantageous embodiments of articulation parts for use with the base plate of Figure 7.
- Figure 9: shows two further advantageous embodiments of a base plate for a femoral component of a trial prosthesis.
- Figure 10: shows two possible embodiments of articulation parts in a front view.
- Figure 11: shows yet a further embodiment of a trial implant according to the invention, (a) in a side-view showing the base plate and the articulation part, and (b) in a front view showing the articulation part.

### Description of embodiments of the invention

The necessary bone cuts for mounting a trial knee prosthesis device according to the invention are schematically described in Figure 3, in a cross-sectional view along the axes of the femur 1 and the tibia 2 in extended position.

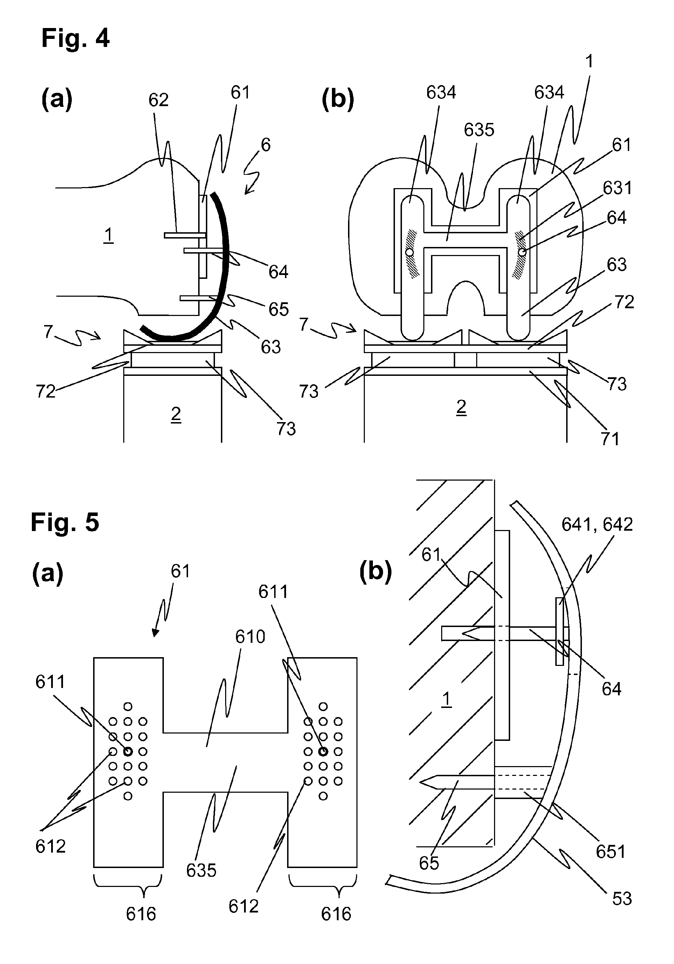

Figure 4 shows a possible embodiment of a trial knee prosthesis device according to the invention, mounted on femur 1 and tibia 2, (a) in a lateral view, and (b) in a frontal view. This particular embodiment is especially suitable for the extension gap first variant of the tibia first technique.

After exposing the knee joint according to standard total knee arthroplasty procedures, in a first step the tibia bone cut D is carried out, already at its final position. In a next step a conservative amount of bone material (12, 12') is removed at the distal end of the femur (cut F) and at the posterior condyle (cut E), namely 2 - 3 mm less than anticipated for the final implant, for example 5 mm. The femoral component 6 and the tibial component 7 of the trial knee prosthesis device according to the invention are then mounted on the prepared femur 1 and tibia 2.

The shown embodiment of a tibial component 7 according to the invention has the outer dimensions of the final tibial implant, and comprises two parts, a base plate part 71, mounted on the proximal cut D of the tibia 2, and an articulation part 72, intended to interact with the femoral component 6. Said articulation part comprises an inlay in the form of a dish that acts as the bearing of the femoral component of the prosthesis, and confines the translational movement of the femoral component along the mediolateral and anteroposterior axis. To reduce friction the inlay is preferably coated with a suitable material, such as high density polyethylene or polyurethane. Since the tibial component does not remain in the body of the patient, also materials may be used that are not suitable for implants, for example polytetrafluoroethylene. The inlay may be fixed, or mobile, to mimic the original tibial joint with its flexible menisci. The actual characteristics of the articulation part of the tibial component should preferably be similar to the final tibial implant.

A resilient element 73 or a similar means is incorporated between the two parts 71, 72 of the tibial component 7, pressing the articulation part 72 against the distal surface of the femoral component 6. The motion of the resilient element 73, the force acting on the resilient element 73, and/or the distance between the base plate part 71 and the articulation part 72 can be measured.

In the embodiment of a tibial component 7 as shown in Figure 4, two resilient elements 73 are arranged on the common base plate 71 in medial and lateral position. Said two elements can be compressed and expanded independently. Compared to an embodiment with only one resilient element, such an approach has the advantage that separate isometricity values can be measured for the medial and the lateral side of the joint. This additional information will allow to identify rotational and varus-valgus misplacements, and thus reduces the necessary steps until an ideal adjustment of the trial prosthesis device is found. Of course one could also use any other suitable number of resilient elements.

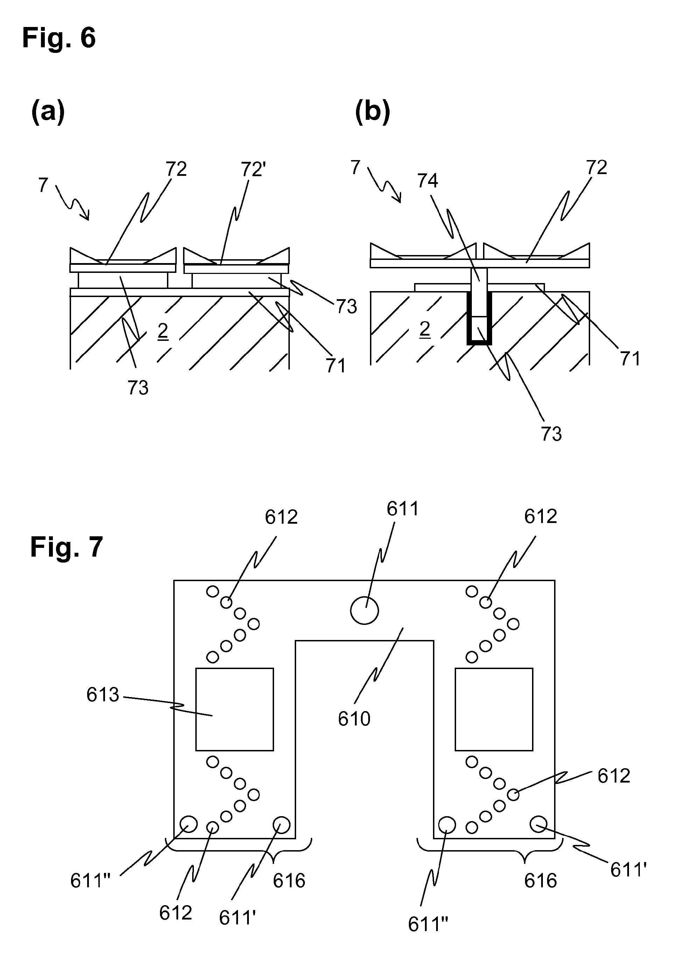

Furthermore it would also be possible to use an articulation part 73 that is divided into two or more subunits, able to independently move from each other and interacting with separate resilient elements. Figure 6(a) schematically shows an embodiment of such a tibial component 7 according to the invention. Another variant of a tibial component 7 is disclosed in Figure 6(b), where the articulation part 72 is mounted on a central stem 74, interacting with a resilient element 73. Said resilient element 73 is arranged in a tube in the tibial bone material 2, and is attached to the base plate 71. By rotation around the stem 74 a rotational misplacement of the femoral component may also be corrected. In the shown embodiment the articulation part is directly connected to the stem 74. In another advantageous embodiment the articulation part 72 and the stem 74 could be connected by a hinge, or could be fixated to each other in a certain angle, in order to correct a varus-valgus misplacement of the femoral component.

The base plate 71 typically has a thickness of about 1 mm, while the articulation part 72 has an overall thickness of about 2 mm. The resilient elements 73 are compressible down to 1 to 2 mm, and expandable up to 10 mm. Hence the overall thickness of the tibial component 7 in the compressed state is 3 to 4 mm, and in the expanded state 10 to 15 mm. If a standard final tibial implant has thickness of 8 to 11 mm, the resilient elements 73 thus can compensate proximodistal movements of up to 4 to 5 mm, which is sufficient to evaluate motional isometricity. The resilient elements 73 should execute a force of at least 100 to 200 N. To adjust the repulsive force the resilient elements may be exchanged.

The femoral component 6 according to the invention comprises a base plate part 61, to be arranged on the distal femoral cut plane F, and an articulation part 63, having the same effective articulation surface as the final femoral implant. The base plate part 61, which is also shown in Figure 5(a) and can be made from metal or any other suitable rigid material, is mounted on the distal femur cut plane F with two anchor pins 62 at medial and lateral position. The positions of the anchor pins 62 are later used as reference points of the cutting guide.

The two anchor pins 62 run through two central pin holes 611 of the base plate 61. It has approximately the shape of the letter H, and a thickness of, for example, 1 mm. A multitude of adjustment pin holes 612 are regularly arranged on a medial and a lateral portion 616 of the base plate part 61, around the two central pin holes 611. In the embodiment of the invention as shown in Figure 5(b), the pin holes 611, 612 are arranged on a grid, which may for example have a grid width of about 1 to 2 mm.

The articulation part 63, which is shown in a closer lateral view in Figure 5(b), consists of two parallel slider elements 634, connected by a crossbeam 635. The slider elements have a thickness of about 1 to 3 mm, a width of about 5 to 15 mm, and have the arcuate sagittal shape of the effective articulation surface of the final femoral implant. The overall width of the articulation part 63 in the mediolateral direction lies between 40 and 70 mm. The exact shape is chosen according to the size and the type/manufacturer of the final femoral implant that eventually should be mounted. Different articulation parts 63 may be combined with one single standard base plate part.

Two second pins 64 are then attached to the distal femur 1 in a lateral and a medial position, in a defined distance to each other, by using a pair of the additional pin holes 64 as a jig. Said two adjustment pins 64, protruding from base plate 61, are arranged to run through two curved long slots 631 located on the slider elements 634, and intended for receiving the second pins 64. The two long slots are part of the same circle, and define a rotational degree of freedom of the articulation part 63 in regard to the base plate 61, around an axis defined by the centre of said circle. To support the articulation part the second pins 64 comprise a support means 641, which is preferably adjustable in height. One possibility is for example a knurled ring, with which the threaded adjustment pin 64 may be driven deeper into the femur 1, thereby adjusting the height of the articulation part 63. To correct the translational displacement of the articulation part 63 along the mediolateral and the anteroposterior axes, the second pins 64 may be shifted along the grid of adjustment pin holes 612, the steps being given by the grid width.

Two supporting pins 65 attached to the articulation part 63, each supporting one of the slider elements 634, are driven into the femur bone, for example with a hammer. Thus each of the slider elements is supported by one adjustment pin 64 and one support pin 65. In the embodiment shown in Figure 5(b), the supporting pin 65 comprises a spacer element 651. Such a spacer element can be adjusted in their length, in order to correct the proximodistal placement of the articulation part 63. If the spacer elements 65 and the support means 641 are chosen appropriately, also a tilt angle of the articulation part relative to the femur plane can be adjusted.

The articulation part 63 is fixed with the pins 62 and 65 on the base plate 63, and is then adjusted in rotation, valgus-varus tilt, and translational displacement, to find the most isometric position. The surgeon will then move the knee joint through the complete range of motion. Since the resilient element 73 of the tibial component 7 according to the invention counteracts with the tension of the ligaments, a constant ligament tension over the whole range of flexion is achieved. The isometricity of this motion is measured as a function of the flexion angle. The deviation from perfect isometricity should remain within an acceptable range of 1 to 2 mm. A not yet ideal positioning of the femoral component 6 will result in an increased motion of the resilient element 73 and the articulation part 72 of the tibial component 7. The tibial component 7 may allow the correction of larger deviations, of up to, for example, 5 mm. If the deviation is even larger, the ligaments will either loosen, and the force acting on the resilient element drops to zero, or the joint will be blocked. In such a case adjustment shims may be placed or removed under the tibial component 7 or the femoral component 6.

If the trial prosthesis behaves isometrically, the ideal position of the prosthesis is found. If not, the following adjustments to the femoral component 6 can be made:
f) distal and proximal translation, which affects the extension gap
g) increasing or decreasing the size of the femoral component, which affects the flexion gap
h) anterior and posterior translation, which affects the flexion gap
i) rotation in the flexion state, which affects the flexion gap
j) varus-valgus alignment, which affects the extension gap

This can be achieved by rotating the articulation part 63 in regard to the base plate part 61 (plus/minus 5 to 10 °), and by a proximodistal, anteroposterior, and/or mediolateral translation (up to 3 mm) of the articulation part 63 in regard to the base plate part 61. To simulate a next larger or smaller final prosthesis size, or even a different prosthesis type, only the articulation part 63 has to be replaced.

Adjustments are repeated until an isometric motion within the accepted range of deviation is achieved. Only then, when the definite position, orientation, and size of the prosthesis are determined, the final, irreversible femur bone cuts are carried out, and the final femoral and tibial implants are mounted. The position of these final cuts A, B, C are determined based on the resulting orientation of the articulation part 63 in view of the base plate part 61, the anchor pins 62 acting as the positional reference after the removal of the base plate. The positions may be obtained from look-up tables, or may be calculated by a computer. The cuts are then carried out with the appropriate cutting guides. Many different types of cutting guides for surgical saws are known from the state of the art.

A further possible embodiment of a base plate for a femoral component of a trial prosthesis is shown in Figure 7. A medial and a lateral portion 616 of the base plate 61 are connected by a cross beam 610. A first central anchor pin hole 611 is arranged on the cross beam 610, through which a first anchor pin (not shown) is driven into the distal femur. The base plate 61 can then be rotated by the user around the rotation axis defined by the first anchor pin. When the appropriate orientation of the base plate is found, a second anchor pin and preferably a third anchor pin (not shown) are driven into the femur through a pair of second anchor pin holes 611' arranged on the two portions 616 of the base plate 61, and the base plate is locked on the femur. When later the base plate has to be rotated to correct a rotational misplacement, the second and third pins can be removed, and another pair of second anchor pin holes 611'' can be used to fasten the base plate. This approach has the advantage that for small angular corrections the second and third anchor pins do not have to be set too close to the previous hole in the distal femur.

Two groups of adjustment pin holes 612 are arranged on both the distal and the lateral portion 616 of the base plate 61. A suitable articulation part 63 can then be fastened to the distal femur by driving two adjustment pins 64 attached to each slider element 634 into the femur, through pairs of holes 612. The mediolateral and anteroposterior position of the articulation part can the be corrected by shifting the slider elements to another pair of adjustment pin holes 612.

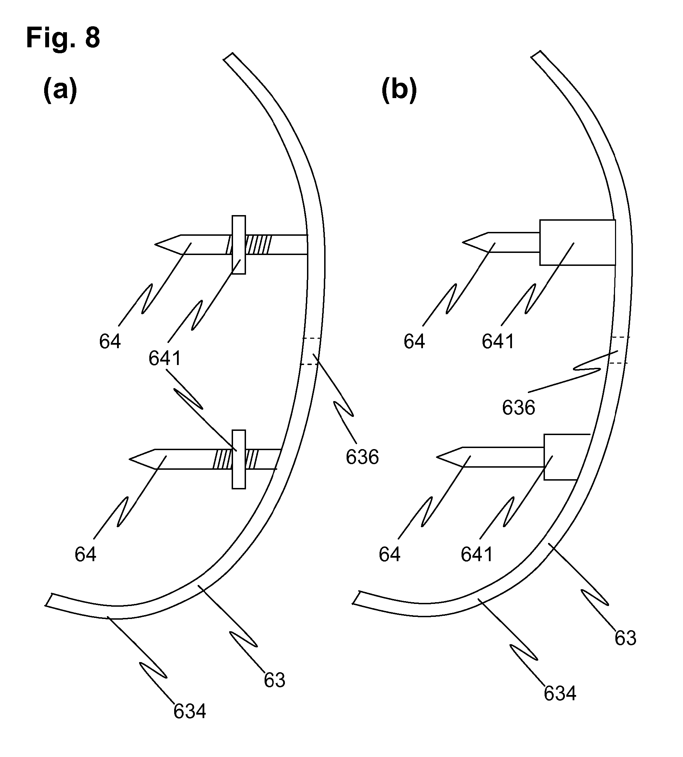

Figure 8 depicts two possible embodiments of an articulation part 63 for use with a base plate 61 as shown in Figure 7. In both embodiments two adjustment pins 64 are attached to a slider element 634. In Figure 8(a) a portion of every pin 64 is threaded, and a knurled ring is rotatably arranged as a supporting means 641 on the pin 64. To adjust the height of the articulation part, the knurled ring is moved to the appropriate position, and the pins 64 are driven into the distal femur through the pin holes 612, until the supporting means 641 is abut to the femur. In Figure 8(b) the same goal is achieved by spacer elements 641 in the form of sockets placed on the pins 64 in a friction-locked manner. The height can be adjusted by exchanging the spacer elements 641.

A hole is located in the centre of the slider element 634, which acts as a drill guide 636. When the most isometric position of the trial implant is found, the surgeon drills a hole into the distal femur below each slider element 634, guided by the drill guide 636. In the base plate 61, an appropriate gap 613 is arranged on both sides, in order to allow a driller to reach the femur. The two drilled holes then act as a positional reference for the cutting guide, similar to the anchor pin holes in the embodiment of Figure 7.

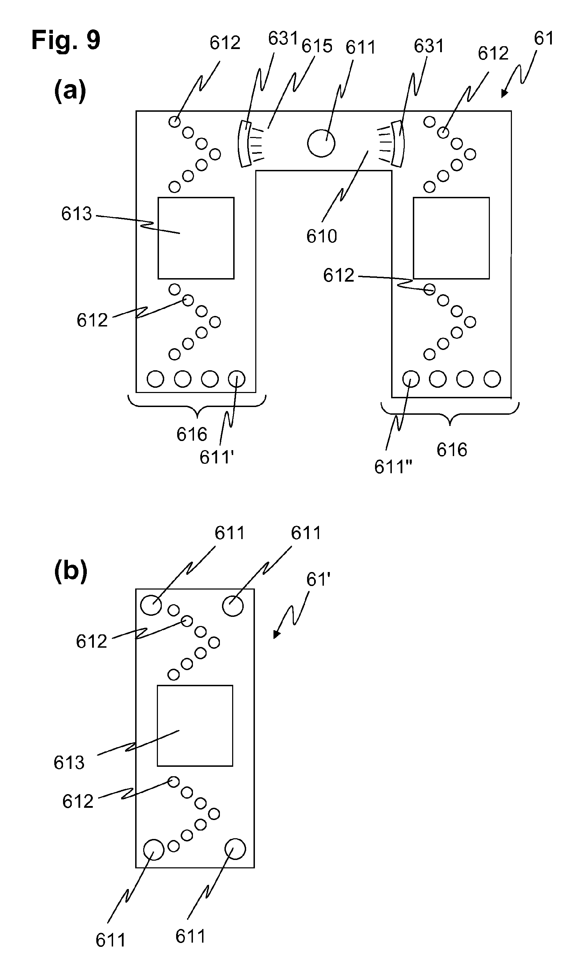

Another embodiment of a base plate 61 is shown in Figure 9(a), which is particularly advantageous in connection with an articulation part as shown in Figure 8. Again a medial and a lateral portion of the base plate 61 are connected by a cross beam 610. On both portions 616 a gap 613 for drilling the reference holes is arranged. In the centre of the cross beam 610 a first anchor pin hole 611 is arranged, through which a first anchor pin (not shown) is driven into the femur bone. Two arcuate long slots 631 are arranged on both sides of the first anchor pin hole 611. The two long slots 631 are part of the same circle, with a centre coinciding with the first anchor pin hole 611. In one or both of the long slots 631 a smaller pin can be placed, which acts as an indicator, showing the rotation angle of the base plate 61 around the first anchor pin.

A surgeon will rotate the base plate until a suitable orientation of the base plate 61 in relation to the femur is found. Then he will drive a second and, advantageously, a third anchor pin (not shown) through one of the second and third anchor pin holes 611', 611'' on the two portions of the base plate 61. If later the base plate has to be rotated by a few degree to correct a rotational misplacement of the trial prosthesis, another second and/or third anchor pin hole 611', 611'' can be used to fasten the base plate to the femur, without having the pins to close to the previous pin holes.

The adjustment pin holes 612 for accommodating the adjustment pins 64 of the articulation parts 63 are arranged in two groups on both portions of the base plate, in a V-shaped pattern. Of course, any other suitable pattern, e.g. a grid, may be applied.

In the previously discussed examples of base plates, the base plate has been realized as one single unit. However, it is also possible to embody the base plate with two separate subunits. Such a subunit 61' is depicted in Figure 9(b). The subunit comprises two groups of adjustment pin holes 612, and four anchor pin holes 611. In the centre of the subunit 61' a gap 613 giving access to the distal femur plane is arranged.

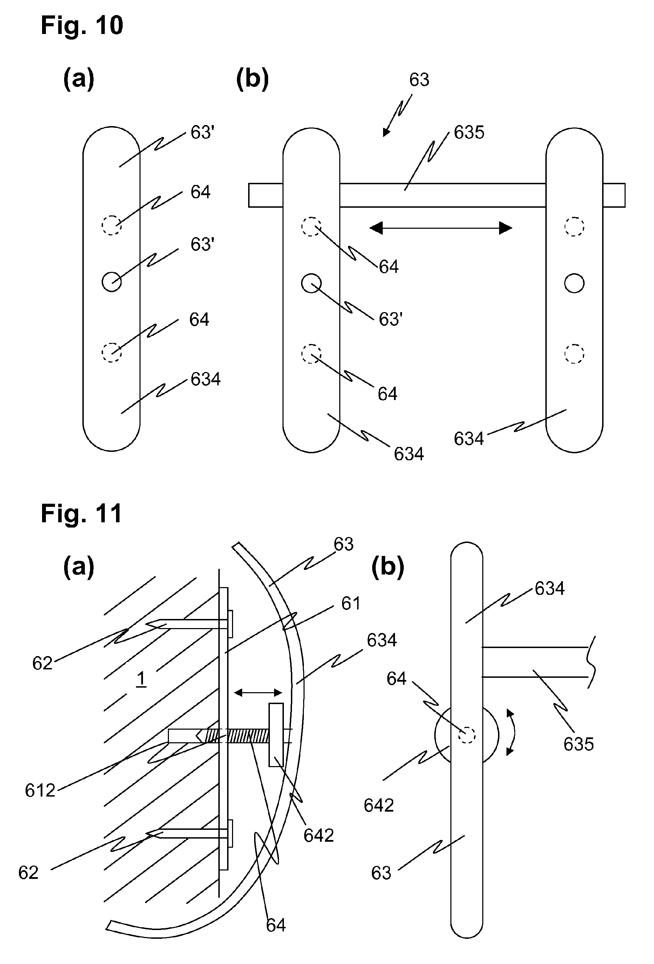

A surgeon can arrange two subunits 61' of a base plate independently from each other, thus having more flexibility in adapting the trial prosthesis. Preferably the two subunits are identical. To make use of the improved flexibility, the articulation part 63 should preferably also be flexible in a certain range. Figure 10 depicts two advantageous embodiments of articulation parts 63 that are particularly suitable for use with a two part base plate as shown in Figure 9(b).

One possibility is to separate the articulation part of the trial prosthesis into two separate subunits, in analogy to the base plate in Figure 9(b). Figure 10(a) shows a front-view of such a separate subunit 63', which comprises only the slider element 634. It thus corresponds to one single condyle of the natural femur. The subunit 63' corresponds to the slider elements 634 of the articulation parts in Figure 8 (a) and (b).

Instead of having two separate subunits 63', the slider elements may be movably arranged on a cross beam. Such a variant is shown in Figure 10(b). Two identical slider elements 634 are slideably arranged on a cross beam 635, and can be fastened in a certain distance to each other with fastening means (not shown). In a preferred embodiment, also the angle between the slider elements 634 and the cross beam 635 can be varied within a certain range.

The same approach can also be used for the base plate. In such an embodiment two separate subunits 61' of a base plate would be slideably connected with a cross beam.

Yet another embodiment of a femoral trial prosthesis according to the invention is shown in Figure 11, (a) in a side-view showing the base plate 61 and the articulation part 63, and (b) in a front-view showing the articulation part 63. In this particular example a threaded adjustment pin 64 is rotatably attached to the slider element 634. The external thread of the pin interacts with an internal thread of an adjustment pin hole 612 of the base plate 61. By turning a knurled ring attached to the pin 64, the pin 64, and thus also the articulation part 63 can be moved in proximodistal direction. If the two slider elements 634 are not connected by a cross beam, an additional means must be applied to fix the rotational orientation of the slider element, such as for example an additional pin.

The present invention is not to be limited in scope by the specific embodiments described herein. Indeed, various modifications of the present invention, in addition to those described herein, will be apparent to those skilled in the art from the foregoing description and accompanying drawings. Thus, such modifications are intended to fall within the scope of the appended claims. Additionally, various references are cited throughout the specification, the disclosures of which are each incorporated herein by reference in their entirety.

In the following some specially preferred embodiments and variants of the invention are defined:

A preferable embodiment of a femoral component (6) of an adjustable trial knee prosthesis device according to the invention comprises a base plate part (61), adapted for the attachment to a distal end (E, F) of a femur (1); an articulation part (63), adapted for the interaction with a tibial component (7) of a knee prosthesis device over essentially the whole range of natural bending angles of a knee joint; and adjustment means for adjusting the translational and/or rotational displacement of the articulation part (63) in regard to the base plate part (61).

Preferably the articulation part (63) of such a femoral component comprises two slider elements (634) that are connected by at least one crossbeam (635), the slider elements (634) being adapted for the interaction with a tibial component (7) of a knee prosthesis device. More preferably the slider elements (634) are slideably moveable on the at least one crossbeam (635).

In yet another preferable embodiment of a femoral component according to the invention, the articulation part (63) comprises two separate subunits (63'), each comprising a slider element (634.

The adjustment means of the above mentioned femoral components comprise one or more spacer elements (651) arranged between the base plate part (61) and the articulation part (63); or at least one adjustment pin (64) is attached to the slider element (634), interacting with an adjustment pin hole (612) of the base plate (61). In the latter case the femoral component the at least one adjustment pin (64) preferably comprises a supporting means (641). A particularly preferable supporting means (641) is an exchangeable spacer element arranged on the adjustment pin (64), or a ring that is translationally movable along a threaded portion of the adjustment pin (64).

In an advantageous embodiment of the femoral component according to the invention the adjustment means comprise two pins (64) attached to the base plate part (61), and a curved long slot (631) on each of the two slider elements (634), wherein said two pins (62) protrude into said curved long slots (634), both lying on a common circle.

In another preferable embodiment an adjustment pin (64) is rotatably connected to a slider element (634), wherein a threaded portion of the pin (64) interacts with an inner thread of an adjustment pin hole (612) of the base plate (61), or with the femur bone (1).

Furthermore it is preferred that the articulation part comprises at least one drill guide hole (636), advantageously in combination with at least one gap (613) arranged on the base plate (61), giving access to a distal femur (1) on which the base plate is arranged.

It is also preferred that the base plate part (61) comprises a multitude of adjustment pin holes (612), and at least one anchor pin hole (611,611',611'').

The base plate part (61) of the femoral component according to the invention may comprise two portions (616), connected with at least one cross beam (610); or may comprise two separate subunits (61').

A preferable embodiment of a tibial component (7) of an adjustable trial knee prosthesis device according to the invention comprises a base plate part (71), adapted for the attachment to a proximal end of a tibia; an articulation part (72, 72'), adapted for the interaction with a femoral component (6) of a knee prosthesis device; and means for providing a repulsive force between said base plate part (71) and said articulation part (72, 72'); wherein the distance between the base plate part (71) and the articulation part (72, 72') is variable within a certain range.

Preferably such a tibial component comprises means for determining the distance between the base plate part (71) and the articulation part (72, 72'), and/or the repulsive force between the base plate part (71) and the articulation part (72, 72').

Advantageously the means of such a tibial component for providing a repulsive force between the base plate part (71) and the articulation part (72, 72') is a resilient element (73), and preferably a spring element. Said resilient element (73) is preferably arranged between the base plate part (71) and the articulation part (72, 72'),; or on the side of the base plate part (71) opposite to the articulation part (72).

In a preferred embodiment the angle between articulation part (72) and base plate part (71) can be adjusted within a certain range. In another preferred embodiment the articulation part (72) is supported by a hinge bearing.

An adjustable trial knee prosthesis device according to the invention with a femoral component (6) and a tibial component (7), adapted to interact with each other, comprises a base plate part (61), adapted for the attachment to a distal end (E, F) of a femur (2); an articulation part (63), adapted for the interaction with the tibial component (7) over essentially the whole range of natural bending angles of a knee joint; and adjustment means for adjusting the translational and/or rotational displacement of the articulation part (63) in regard to the base plate part (61).

Preferably the tibial component (7) of such an adjustable trial knee prosthesis device comprises a base plate part (71), adapted for the attachment to a proximal end of a tibia (2); an articulation part (72, 72'), adapted for the interaction with a femoral component (6) of a knee prosthesis device; and means for providing a repulsive force between said base plate part (71) and said articulation part (72, 72'); wherein the distance between the base plate part (71) and the articulation part (72, 72') is variable within a certain range.

In another advantageous embodiment the tibial component (7) of the adjustable trial knee prosthesis device comprises means for measuring a force acting in proximodistal direction on an articulation part (72, 72').

It is preferred that the adjustable trial knee prosthesis device comprises a femoral component (6) and/or a tibial component (6) as discussed above.

In a method for positioning a knee prosthesis according to the invention, (a) in a first step a femoral component (6) of a trial knee prosthesis device with a an articulation part (63) and a base plate part (61) is mounted on a distal end (E, F) of a femur (1), and a tibial component (7) of a trial knee prosthesis device with an articulation part (72, 72') and a base plate part (71) is mounted on a proximal end (D) of a corresponding tibia (2); the tibial component (7) having a variable distance between its base plate part (71) and its articulation part (72, 72'), and being subject to a repulsive force between its base plate part (71) and its articulation part (72, 72'); and the femoral component (7) comprising means for adjusting the translational and/or rotational displacement of its articulation part (63) in regard to its base plate part (61); (b) in a second step the repulsive force acting on the tibial component (7) and/or the position of its articulation part (72, 72') in regard to its base plate part (71) is measured for a multitude of different knee flexion angles; (c) in a third step the results found during the second step are utilized to correct the translational and/or rotational displacement of the articulation part (63) of the femoral component (6) in regard to its base plate part (61), and/or to correct the dimensions of the articulation part (63) by replacing it by an-other articulation part with different dimensions; (d) the second step and third step are repeated until an isometric knee motion is reached, during which the measured repulsive force acting on the tibial component (7) and/or the determined position of its articulation part (72, 72') in regard to its base plate part (71) do not deviate more than a predefined value from a certain average value; (e) in a forth step the appropriate-ate femoral cutting positions, the component sizes of the final prosthesis device, and their orientation in regard to the tibia and the femur are determined, based on the found adjustments of the trial prosthesis device and/or the values determined in the second steps.

In a preferable variant of such a method in the second step the repulsive force acting on the tibial component (7) and/or the determined position of its articulation part (72, 72') in regard to its base plate part (71) are measured over the complete range of natural knee flexion angles.

It is preferred that such a method is carried out using an adjustable trial knee prosthesis device, a femoral component (6) and/or a tibial component (6) as discussed above.

### List of Reference Numerals

- 1: femur
- 11, 11', 11'': removed bone material
- 12, 12': removed bone material
- 2: tibia
- 21: removed bone material
- 3: fibula
- 41: Medial collateral ligament
- 42: Lateral collateral ligament
- 51: flexion gap
- 52: extension gap
- 6: femoral component of trial prosthesis
- 61, 61': base plate part
- 610: cross beam
- 611, 611', 611'': anchor pin hole
- 612: adjustment pin hole
- 613: drill gap
- 615: angle scale
- 616: portion of base plate
- 62, 62', 62'': anchor pin
- 63, 63': articulation part
- 631: long slot
- 634: slider element
- 635: cross beam
- 636: drill guide
- 64: adjustment pin
- 641: supporting means, spacer element
- 642: knurled ring
- 65: support pin
- 651: spacer element
- 7: tibial component of trial prosthesis
- 71: base plate part
- 72, 72': articulation part
- 73: resilient element
- 74: stem
- A: anterior femoral bone cut
- B: posterior femoral bone cut
- C: distal femoral bone cut
- D: proximal tibial bone cut
- E: conservative posterior condyle bone cut
- F: conservative distal femoral bone cut
- I: extended position, 0°
- II: flexed position, 90°

## Claims

1. A femoral component (6) of an adjustable trial knee prosthesis device, comprising a base plate part (61), adapted for the attachment to a distal end (E, F) of a femur (1); an articulation part (63), adapted for the interaction with a tibial component (7) of a knee prosthesis device over essentially the whole range of natural bending angles of a knee joint; and adjustment means for adjusting the translational and/or rotational displacement of the articulation part (63) in regard to the base plate part (61).

2. The femoral component according to claim 1, **characterized in that** the articulation part (63) comprises two slider elements (634) that are connected by at least one crossbeam (635), the slider elements (634) being adapted for the interaction with a tibial component (7) of a knee prosthesis device.

3. The femoral component according to claim 1, **characterized in that** the articulation part (63) comprises two separate subunits (63'), each comprising a slider element (634.

4. The femoral component according to any of claims 1 to 3, **characterized in that** the adjustment means comprise one or more spacer elements (651) arranged between the base plate part (61) and the articulation part (63).

5. The femoral component according to any of claims 1 to 3, **characterized in that** at least one adjustment pin (64) is attached to the slider element (634), interacting with an adjustment pin hole (612) of the base plate (61), and preferably comprising a supporting means (641).

6. The femoral component according to any of claims 1 to 5, **characterized in that** the articulation part comprises at least one drill guide hole (636).

7. The femoral component according to any of claims 1 to 6, **characterized in that** the base plate part (61) comprises a multitude of adjustment pin holes (612), and at least one anchor pin hole (611, 611', 611'').

8. A tibial component (7) of an adjustable trial knee prosthesis device, comprising a base plate part (71), adapted for the attachment to a proximal end of a tibia; an articulation part (72, 72'), adapted for the interaction with a femoral component (6) of a knee prosthesis device; and means for providing a repulsive force between said base plate part (71) and said articulation part (72, 72'); wherein the distance between the base plate part (71) and the articulation part (72, 72') is variable within a certain range.

9. The tibial component according to claim 8, **characterized by** means for determining the distance between the base plate part (71) and the articulation part (72, 72'), and/or the repulsive force between the base plate part (71) and the articulation part (72, 72');

10. The tibial component according to claim 8 or 9, **characterized in that** the means for providing a repulsive force between said base plate part (71) and said articulation part (72, 72') is a resilient element (73), particularly a spring element; the resilient element (73) preferably being arranged between the base plate part (71) and the articulation part (72, 72'), or on the side of the base plate part (71) opposite to the articulation part (72).

11. The tibial component according to any of claims 8 to 10, **characterized in that** the angle between articulation part (72) and base plate part (71) can be adjusted within a certain range.

12. An adjustable trial knee prosthesis device with a femoral component (6) and a tibial component (7), adapted to interact with each other, **characterized in that** the femoral component (6) comprises a base plate part (61), adapted for the attachment to a distal end (E, F) of a femur (2); an articulation part (63), adapted for the interaction with the tibial component (7) over essentially the whole range of natural bending angles of a knee joint; and adjustment means for adjusting the translational and/or rotational displacement of the articulation part (63) in regard to the base plate part (61).

13. The adjustable trial knee prosthesis device according to claim 12, **characterized in that** the tibial component (7) comprises a base plate part (71), adapted for the attachment to a proximal end of a tibia (2); an articulation part (72, 72'), adapted for the interaction with a femoral component (6) of a knee prosthesis device; and means for providing a repulsive force between said base plate part (71) and said articulation part (72, 72'); wherein the distance between the base plate part (71) and the articulation part (72, 72') is variable within a certain range.

14. The adjustable trial knee prosthesis device according to claim 12, **characterized in that** the tibial component (7) comprises means for measuring a force acting in proximodistal direction on an articulation part (72, 72').

15. The adjustable trial knee prosthesis device according to any of claims 12 to 14, with a femoral component (6) according to any of claims 1 to 7, and/or with a tibial component (6) according to any of claims 8 to 11.

16. A method for positioning a knee prosthesis, wherein (a) in a first step a femoral component (6) of a trial knee prosthesis device with a an articulation part (63) and a base plate part (61) is mounted on a distal end (E, F) of a femur (1), and a tibial component (7) of a trial knee prosthesis device with an articulation part (72, 72') and a base plate part (71) is mounted on a proximal end (D) of a corresponding tibia (2); the tibial component (7) having a variable distance between its base plate part (71) and its articulation part (72, 72'), and being subject to a repulsive force between its base plate part (71) and its articulation part (72, 72'); and the femoral component (7) comprising means for adjusting the translational and/or rotational displacement of its articulation part (63) in regard to its base plate part (61); (b) in a second step the repulsive force acting on the tibial component (7) and/or the position of its articulation part (72, 72') in regard to its base plate part (71) is measured for a multitude of different knee flexion angles; (c) in a third step the results found during the second step are utilized to correct the translational and/or rotational displacement of the articulation part (63) of the femoral component (6) in regard to its base plate part (61), and/or to correct the dimensions of the articulation part (63) by replacing it by an-other articulation part with different dimensions; (d) the second step and third step are repeated until an isometric knee motion is reached, during which the measured repulsive force acting on the tibial component (7) and/or the determined position of its articulation part (72, 72') in regard to its base plate part (71) do not deviate more than a predefined value from a certain average value; (e) in a forth step the appropriate femoral cutting positions, the component sizes of the final prosthesis device, and their orientation in regard to the tibia and the femur are determined, based on the found adjustments of the trial prosthesis device and/or the values determined in the second steps.

17. The method according to claim 16, **characterized in that** in the second step the repulsive force acting on the tibial component (7) and/or the determined position of its articulation part (72, 72') in regard to its base plate part (71) are measured over the complete range of natural knee flexion angles.
